# EUROPEAN PATENT APPLICATION

(11) **EP 2 578 210 A1**
(43) Date of publication of application: **10.04.2013**
(21) Application number: 11184003.9
(22) Date of filing: 05.10.2011
(51) Int. Cl.: A61K 31/00, A61K 31/352

(54) **Rottlerin for the treatment of pulmonary hypertension and related diseases and disorders**

(71) Applicant: ATB Innovation Ltd., 72076 Tuebingen (DE)
(72) Inventor: Bachmann, Alexander, 72076 Tuebingen (DE)
(74) Representative: Isarpatent

(57) **Abstract**

The inventions discloses a pharmaceutical composition comprising rottlerin or a pharmaceutically acceptable salt thereof or one or more of its derivatives or a pharmaceutically acceptable salt thereof said derivative having the formula 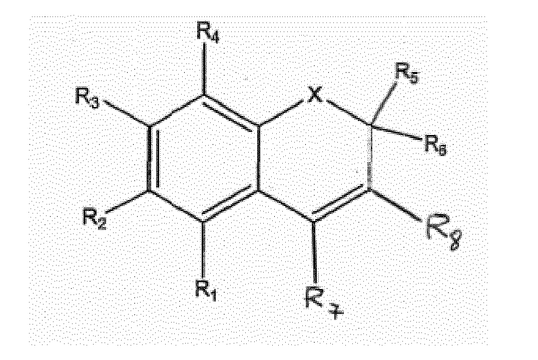 wherein **X** is selected from the group consisting of CH₂, O, N, P, and S; **R1** and **R3** are selected from the group consisting of H, OH, NH₂, SH, SO, S-NH-R, NH-R (R selected from the group consisting of alkyl, alkenyl, alkinyl, aryl, phenyl, benzyl, ester, ether, carbonyl, carboxyl, F, Cl, Br, I, H), alkyl, alkenyl, alkinyl, aryl, phenyl, benzyl, ester, ether, carbonyl, carboxyl, F, Cl, Br, I and H; **R2** is selected from the group consisting of ethanone, acetyl, alkyl, alkenyl alkinyl, aryl, phenyl, benzyl, ester ether, carbonyl, carboxyl, F, Cl, Br, I and H; **R4** is CO-[(E)CHCH]n-Ph, CN-[(E)CHCH]n-Ph, or COOZ, wherein Z is selected from the group consisting of alkenyl, aryl, and alkyl, alkyl, alkenyl, alkinyl, aryl, phenyl, benzyl, ester, ether, carbonyl, carboxyl, F, Cl, Br, I and H; **R5, R6, R7 and R8** are selected from the group consisting of methyl, H, P, OH, NH₂, SH, SO, S-NH-R, NH-R (R selected from the group consisting of alkyl, alkenyl, alkinyl, aryl, phenyl, benzyl, ester, ether, carbonyl, carboxyl, F, Cl, Br, I, H), alkyl, alkenyl, alkinyl, aryl, phenyl, benzyl, ester, ether, carbonyl, carboxyl, F, Cl, Br, I and H, for use in a method of preventing or treating pulmonary hypertension.

## Description

### Technical Field

This invention relates to methods and compositions for the treatment of pulmonary hypertension and related diseases and disorders.

### Background Art

Pulmonary hypertension (PH), or pulmonary arterial hypertension (PAH), is a disease characterized by increased pulmonary artery pressure and pulmonary vascular resistance. The disease is defined by a mean pulmonary artery pressure (PAP) > 25mm Hg at rest or > 30mg Hg with exercise.

Pulmonary hypertension results from constriction, or stiffening, of the pulmonary arteries that supply blood to the lungs. Consequently, it becomes more difficult for the heart to pump blood forward through the lungs. This stress on the heart leads to enlargement of the right heart and eventually fluid can build up in the liver and other tissues, such as in the legs.

In the conventional classification, pulmonary hypertension, which is also called pulmonary arterial hypertension, is divided into two main categories; 1) primary pulmonary hypertension (not caused by any other disease or condition); and 2) secondary pulmonary hypertension (caused by another underlying condition). Secondary pulmonary hypertension is much more common than primary pulmonary hypertension.

A newer classification of this condition is based on the main underlying cause of pulmonary hypertension. This system classifies the condition based on whether it is due to:
- Pulmonary hypertension from a variety of causes, some known and others unknown, such as:
   o drug-induced pulmonary artery hypertension,
   o pulmonary artery hypertension associated with collagen vascular diseases, HIV, and schistosomiasis (a parasitic infection), and o pulmonary arterial hypertension of unknown cause (idiopathic pulmonary arterial hypertension)
- Pulmonary arterial hypertension from left-sided heart disease, often referred to as pulmonary venous hypertension, including thickening of the heart muscle, decreased heart pump function, and disorders of the heart valves
- Pulmonary arterial hypertension associated with lung disease and or persistent drop in oxygen levels (hypoxia) including:
   o COPD (chronic obstructive pulmonary disease),
   o sleep apnea,
   o pulmonary fibrosis, and
   o living at high elevations
- Pulmonary arterial hypertension from blood clotting disorders that deliver clots to the lung (pulmonary emboli) or are formed directly in the lung arteries (pulmonary thrombosis), also known as chronic thromboembolic pulmonary hypertension;
- Pulmonary arterial hypertension from miscellaneous causes with unclear multifactorial mechanisms; including diseases such as:
   o sarcoidosis,
   o tumor obstruction,
   o metabolic disorders (glycogen storage disease), and
   o kidney failure

The treatment for pulmonary hypertension depends on the underlying cause.

If left sided heart failure is the primary problem, then adequate treatment of the left heart failure by a cardiologist is the main stray of treatment.

In cases where hypoxia (low oxygen levels) due to any chronic lung disease, such as COPD, is the cause, then providing oxygen and appropriately treating the underlying lung disease by a lung doctor (pulmonologist) is the first step in treatment.

In conditions, such as scleroderma, which often can cause pulmonary hypertension, a rheumatologist is involved in the treatment program.

Anticoagulation (thinning the blood) may be an option for treatment if the main underlying cause is thought to be recurrent blood clot (chronic thromboembolic pulmonary hypertension). As indicated in the previous section, referral to a specialty center may be warranted for a possible surgical removal of blood clot (thromboendarterectomy).

For patients with primary pulmonary hypertension (those with no underlying cause), more advanced therapy may be attempted. These drugs have complex mechanisms, but in general they work by dilating (opening up) the pulmonary arteries and, therefore, by reducing the pressure in these blood vessels and some help prevent the excessive overgrowth of tissue in the blood vessels (that decrease remodeling of the vessels, as described above).

There are three major classes of drugs used to treat idiopathic pulmonary hypertension and pulmonary hypertension associated with collagen vascular diseases: 1. prostaglandins; 2. phosphodiesterase type 5 inhibitor; and 3. endothelium antagonists.
1. Prostaglandins such as epoprostenol (Flolan), treprostinil (Remodulin), iloprost (Ventavis). These drugs are very short-acting and often must be given intravenously or inhaled on a very frequent or continuous basis.
2. Phosphodiesterase type 5 inhibitors such as sildenafil (Revatio, Viagra) and tadalafil (Adcirca, Cialis) are somewhat less effective than the prostaglandins, but are easily administered one to three times per day by mouth. (The dosing is much different when these drugs are used for erectile dysfunction.)
3. Endothelium antagonists are the newest medications used for this condition. These include bosentan (Tracleer) and ambrisentan (Letairis). These medications are also given by mouth one to two times per day.

Endothelin receptor antagonists work by binding to the ETA and/or ETB receptor sites in the endothelium and vasculature smooth muscle, thereby preventing the neurohormone endothelin-1 (ET-1) from binding to these same receptor sites and triggering vasoconstriction. An example of an ERA is bosentan (TRACLEER(R)).

In rare cases, calcium channel blockers may be of benefit.

Currently, research is investigating the best ways to combine these medications for the optimal clinical outcomes.

Other methods of treating PH have been investigated. For example, selective serotonin reuptake inhibitors (SSRIs) reportedly reverse PH in rats. These compounds, which are widely used to treat depression, affect the reuptake of the neurotransmitter serotonin (5-HT). Further drugs currently used to treat PH include pulmonary vasodilators like nitric oxide and inhibitors of platelet aggregation.

It should be noted that these medications are extremely expensive, costing thousands of thousands of Euros per year. The companies that manufacture these medications often have programs to assist in funding.

These more advanced therapies have also been used for other forms of pulmonary hypertension, however, no clinical studies have yet confirmed benefits in these situations.

It is worth mentioning that regardless of the cause of pulmonary hypertension, supplemental oxygen and diuretics may play an important role in relieving the symptoms of pulmonary hypertension of any cause. Low oxygen in the atmosphere causes low blood oxygen levels and aggravates pulmonary hypertension. Therefore, patients with pulmonary hypertension may benefit from breathing supplemental oxygen, especially during air travel or traveling to high altitude destinations.

Despite advances in various treatments, there is no cure for pulmonary hypertension.

Generally, the prognosis of pulmonary varies depending on the underlying condition that is causing it. For idiopathic or familial pulmonary hypertension, the overall prognosis depends on the severity and whether treatment was instituted. The statistics show a survival of about 3 years in idiopathic pulmonary hypertension without any therapy. Some of the other factors may indicate even poorer prognosis which include severe symptoms, age of onset greater than 45 years, evidence of right sided heart failure, and failure to respond to treatment. For patients with idiopathic pulmonary hypertension who get started on treatment and respond to it, the prognosis is better. Studies are underway to determine optimal treatment regimens.

In spite of the different therapeutic approaches mentioned above the medical need to alleviate the disease burden in pulmonary hypertension is high. It is therefore an object of the present invention to make available pharmaceutical compositions for the preventive or curative treatment of pulmonary hypertension, which overcome some or all of the known disadvantages.

Rottlerin (also called mallotoxin or kamala; see formula 1), is a 5,7-dihydroxy-2,2-dimethyl-6-(2,4,6-trihydroxy-3-methyl-5-acetylbenzyl)-8-cinnamoyl-1,2-chromene, a polyphenol, is a pigmented plant product isolated from *mallotus philippinensis,* used in India in ancient times as a remedy for tapeworm.

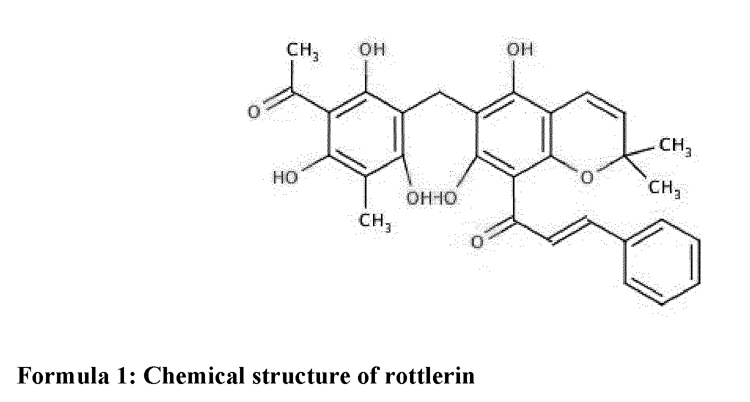

Since 1994, rottlerin has been used as a PKCδ inhibitor based on *in vitro* studies that have shown that the IC₅₀ for PKCδ was 3-6 µM compared to 30-100 µM for the other PKC isozymes, although the selectivity of rottlerin in inhibiting PKC δ isoform has been recently questioned. Furthermore rottlerin can inhibit many kinases and has several biological effects.

Several phloroglucinol derivatives, which are similar to rottlerin in structure, have been demonstrated to have antiinflammatory and antiallergic properties. Moreover, rottlerin can target mitochondria, interfering in the respiratory chain as an uncoupler of oxidative phosphorylation, and activates the large conductance voltage and Ca²⁺ activated K⁺ channel (BK) in human vascular smooth muscle.

In US 2010/298352 rottlerin has been described as a compound that selectively inhibits cancer stem cells; WO 2007/106424 describes rottlerin as useful in the treatment of individuals having cancers with over-activation of the Ras signaling pathway.

JP 2010-043061 claims rottlerin as an anti-helicobacter pylori agent.

WO 2006/060196 discloses that rottlerin and derivatives thereof are potent activators of the BK channel and that hypertension and related disorders can be treated or prevented via regulation of the BK channel using rottlerin. In this closest prior art the use of rottlerin for the treatment of pulmonary hypertension is not mentioned.

### Disclosure of Invention

Provided herein is a composition and a method of preventing or delaying onset of PH. The active pharmaceutical agent disclosed herein may be administered as part of a combination treatment with one or more other compounds. The subject may be undergoing treatment for another disease and the PH may be secondary to the other disease.

This invention is directed, in part, to compositions and methods of treating pulmonary hypertension and related diseases and disorders, which comprise administering to a patient therapeutically effective amounts of rottlerin (formula 1) or a pharmaceutically acceptable salt thereof or one or more of its derivatives or a pharmaceutically acceptable salt thereof.

Derivates of rottlerin have the following Formula 2 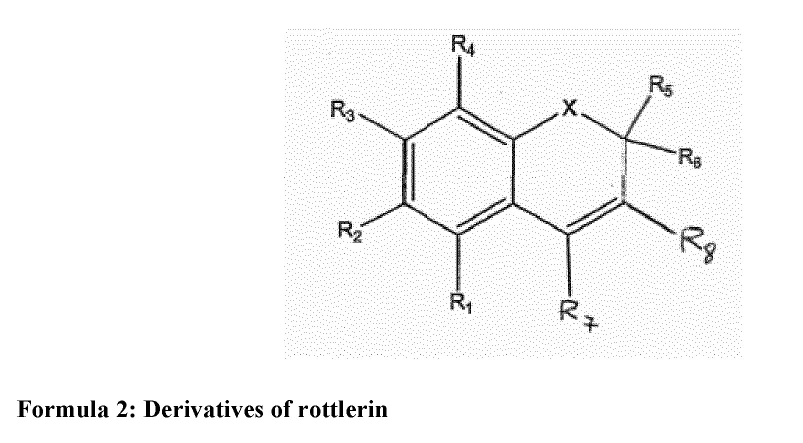 wherein **X** is selected from the group consisting of CH₂, O, N, P, and S; **R1** and **R3** are selected from the group consisting of H, OH, NH₂, SH, SO, S-NH-R, NH-R (R selected from the group consisting of alkyl, alkenyl, alkinyl, aryl, phenyl, benzyl, ester, ether, carbonyl, carboxyl, F, Cl, Br, I, H), alkyl, alkenyl, alkinyl, aryl, phenyl, benzyl, ester, ether, carbonyl, carboxyl, F, Cl, Br, I and H; **R2** is selected from the group consisting of ethanone, acetyl, alkyl, alkenyl alkinyl, aryl, phenyl, benzyl, ester ether, carbonyl, carboxyl, F, Cl, Br, I and H; **R4** is CO-[(E)CHCH]n-Ph, CN-[(E)CHCH]n-Ph, or COOZ, wherein Z is selected from the group consisting of alkenyl, aryl, and alkyl, alkyl, alkenyl, alkinyl, aryl, phenyl, benzyl, ester, ether, carbonyl, carboxyl, F, Cl, Br, I and H; **R5, R6, R7 and R8** are selected from the group consisting of methyl, H, P, OH, NH₂, SH, SO, S-NH-R, NH-R (R selected from the group consisting of alkyl, alkenyl, alkinyl, aryl, phenyl, benzyl, ester, ether, carbonyl, carboxyl, F, Cl, Br, I, H), alkyl, alkenyl, alkinyl, aryl, phenyl, benzyl, ester, ether, carbonyl, carboxyl, F, Cl, Br, I and H.

A treatment of pulmonary hypertension can surprisingly be achieved by the use of a compound of formula 1 or a pharmaceutically acceptable salt thereof and/or a derivative thereof (formula 2).

It is understood that the compound of formula 1 and its pharmaceutically acceptable salts or derivatives can also be present in the form of their pharmaceutically acceptable solvates or in the form of their hydrates.

One embodiment of the present invention encompasses a composition for use in a method of treating or preventing pulmonary hypertension, which method comprises administering to a patient in need thereof therapeutically or prophylactically effective amounts of rottlerin or a pharmaceutically acceptable salt thereof or one or more of its derivatives or a pharmaceutically acceptable salt thereof, alone or in combination with one or more pharmaceutically active ingredients.

A preferred embodiment encompasses a composition for use in a method of treating or preventing pulmonary hypertension, which method comprises administering to a patient in need thereof therapeutically or prophylactically effective amounts of an anticoagulant and rottlerin or a pharmaceutically acceptable salt thereof or one or more of its derivatives or a pharmaceutically acceptable salt thereof.

Another preferred embodiment encompasses a composition for use in a method of treating or preventing pulmonary hypertension, which method comprises administering to a patient in need thereof therapeutically or prophylactically effective amounts of a calcium channel blocker and rottlerin or a pharmaceutically acceptable salt thereof or one or more of its derivatives or a pharmaceutically acceptable salt thereof.

Another preferred embodiment encompasses a composition for use in a method of treating or preventing pulmonary hypertension, which method comprises administering to a patient in need thereof therapeutically or prophylactically effective amounts of a prostaglandine and rottlerin or a pharmaceutically acceptable salt thereof or one or more of its derivatives or a pharmaceutically acceptable salt thereof. The prostaglandine might be selected from the group consisting of alprostadil, epoprostenol, iloprost, and treprostinil.

Another preferred embodiment encompasses a composition for use in a method of treating or preventing pulmonary hypertension, which method comprises administering to a patient in need thereof therapeutically or prophylactically effective amounts of nitric oxide or a nitric oxide precursor or releasing compound, and rottlerin or a pharmaceutically acceptable salt thereof or one or more of its derivatives or a pharmaceutically acceptable salt thereof. The nitric oxide precursor or releasing compound might be selected from the group consisting of dihydralazine mesylate, dihydralazine sulfate, minoxidile, nitric oxide, and sodium nitroprusside.

Another preferred embodiment encompasses a composition for use in a method of treating or preventing pulmonary hypertension, which method comprises administering to a patient in need thereof therapeutically or prophylactically effective amounts of a phosphodiesterase 5 inhibitor and rottlerin or a pharmaceutically acceptable salt thereof or one or more of its derivatives or a pharmaceutically acceptable salt thereof. The phosphodiesterase 5 inhibitor might be selected from the group consisting of avanafil, enoximon, icariin, mirodenafil, milrinon, sildenafil, sildenafil citrate, tadalafil, vardenafil, and udenafil.

Another preferred embodiment encompasses a composition for use in a method of treating or preventing pulmonary hypertension, which method comprises administering to a patient in need thereof therapeutically or prophylactically effective amounts of a diuretic and rottlerin or a pharmaceutically acceptable salt thereof or one or more of its derivatives or a pharmaceutically acceptable salt thereof. The diuretic might be selected from the group consisting of high ceiling loop diuretics like bumetanide, ethacrynic acid, furosemide, torsemide; thiazides such as hydrochlorothiazide; carbonic anhydrase inhibitors like acetazolamide, methazolamide; potassium-sparing diuretics like aldosterone antagonists (such as spironolactone, potassium canreonate) and epithelial sodium channel blockers (such as amiloride, triamterene); calcium-sparing diuretics; osmotic diuretics like mannitol, isosorbide; and low ceiling diuretics.

Another preferred embodiment encompasses a composition for use in a method of treating or preventing pulmonary hypertension, which method comprises administering to a patient in need thereof therapeutically or prophylactically effective amounts of a platelet derived growth factor and rottlerin or a pharmaceutically acceptable salt thereof or one or more of its derivatives or a pharmaceutically acceptable salt thereof. The platelet derived growth factor might be selected from the group consisting of PDGFA, PDGFB, PDGFC, PDGFD, PDGFRα, and PDGFRβ.

Another preferred embodiment encompasses a composition for use in a method of treating or preventing pulmonary hypertension, which method comprises administering to a patient in need thereof therapeutically or prophylactically effective amounts of an endothelium antagonist and rottlerin or a pharmaceutically acceptable salt thereof or one or more of its derivatives or a pharmaceutically acceptable salt thereof. The endothelium antagonist might be selected from the group consisting of ambrisentan, atrasentan, BQ-123, sitaxentan, zibotentan, bosentan, tezosentan, and BQ-788.

Another preferred embodiment encompasses a composition for use in a method of treating or preventing pulmonary hypertension, which method comprises administering to a patient in need thereof therapeutically or prophylactically effective amounts of a selective serotonin reuptake inhibitor (SSRI) and rottlerin or a pharmaceutically acceptable salt thereof or one or more of its derivatives or a pharmaceutically acceptable salt thereof. The SSRI might be selected from the group consisting of citalopram, dapoxetine, escitalopram, fluoxetine, fluvoxamine, indalpine, paroxetine, sertraline, vilazodone, and zimelidine.

Another embodiment of the invention is a composition for use in a method of regulating, treating or preventing membrane excitability or a hyperexcitability disorder in a subject, comprising administering rottlerin or a pharmaceutically acceptable salt thereof or one or more of its derivatives, alone or in combination with one or more pharmaceutically active ingredients, to a subject in need thereof.

In a preferred embodiment the membrane excitability or a hyperexcitability disorder is a skin disease selected from the group consisting of neurodermatitis or atopic eczema.

The route of administration for regulating, treating or preventing membrane excitability or a hyperexcitability disorder is preferably topical or transdermal in a formulation selected from the group consisting of a cream, gel and ointment.

The invention also encompasses pharmaceutical formulations (e.g., single unit dosage forms) comprising rottlerin or a pharmaceutically acceptable salt thereof or one or more of its derivatives or a pharmaceutically acceptable salt thereof alone or in combination with at least one other active pharmaceutical ingredient for the above indicated use. Examples of such other active pharmaceutical ingredients include those described herein, as well as other known by those skilled in the art.

One may administer one other active pharmaceutical ingredient before, after, or at the same time as administering rottlerin or a pharmaceutically acceptable salt thereof or one or more of its derivatives or a pharmaceutically acceptable salt thereof.

Certain pharmaceutical compositions such as, but not limited to, immediate release, modified release (prolonged, delayed, extended release) or depot compositions are single unit dosage forms suitable for systemic, inhalation, oral, mucosal (e.g., nasal, sublingual, vaginal, buccal, or rectal), parenteral (e.g., subcutaneous, intravenous, bolus injection, intramuscular, or intraarterial), transdermal or topical administration to a patient. Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; implants; solutions; patches; aerosols (e.g., nasal sprays or inhalers); gels; liquid dosage forms suitable for oral or mucosal administration to a patient, including suspensions (e.g., aqueous or nonaqueous liquid suspensions, oil-in-water emulsions, or a water-in-oil liquid emulsions), solutions, and elixirs; liquid dosage forms suitable for parenteral administration to a patient; and sterile solids (e.g., crystalline or amorphous solids) that can be reconstituted to provide liquid dosage forms suitable for parenteral administration to a patient.

The formulation should suit the mode of administration. For example, the oral administration of a compound susceptible to degradation in the stomach may be achieved using an enteric coating. Similarly, a formulation may contain ingredients that facilitate delivery of the active ingredient(s) to the site of action. For example, compounds may be administered in liposomal formulations in order to protect them from degradative enzymes, facilitate transport in circulatory system, and affect their delivery across cell membranes.

Similarly, poorly soluble compounds may be incorporated into liquid dosage forms (and dosage forms suitable for reconstitution) with the aid of solubilizing agents, emulsifiers and surfactants such as, but not limited to, cyclodextrins (e.g., α-cyclodextrin, β-cyclodextrin, Captisol(R), and Encapsin(TM), Labrasol(R), Labrafil(R), Labrafac(R), cremafor, and non-aqueous solvents, such as, but not limited to, ethyl alcohol, isopropyl alcohol, ethyl carbonate, ethyl acetate, benzyl alcohol, benzyl benzoate, propylene glycol, 1,3-butylene glycol, dimethyl formamide, dimethyl sulfoxide (DMSO), biocompatible oils (e.g., cottonseed, groundnut, corn, germ, olive, castor, and sesame oils), glycerol, tetrahydrofurfuryl alcohol, polyethylene glycols, fatty acid esters of sorbitan, and mixtures thereof (e.g., DMSO:cornoil).

Poorly soluble compounds may also be incorporated into suspensions using other techniques known in the art. For example, nanoparticles of a compound may be suspended in a liquid to provide a nanosuspension.

The composition, shape, and type of a dosage form will typically vary depending with use. For example, a dosage form used in the acute treatment of a disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease. How to account for such differences will be apparent to those skilled in the art.

The expression "pulmonary hypertension" as used herein comprises different forms of pulmonary hypertension. Nonlimiting examples, which may be mentioned in this connection are idiopathic pulmonary arterial hypertension; familial pulmonary arterial hypertension; pulmonary arterial hypertension associated with collagen vascular disease, congenital systemic-to-pulmonary shunts, portal hypertension, HIV infection, drugs or toxins; pulmonary hypertension associated with thyroid disorders, glycogen storage disease, Gaucher disease, hereditary hemorrhagic telangiectasia, hemoglobinopathies, myeloproliferative disorders or splenectomy; pulmonary arterial hypertension associated with pulmonary capillary hemangiomatosis; persistent pulmonary hypertension of the newborn; pulmonary hypertension associated with chronic obstructive pulmonary disease, interstitial lung disease, hypoxia driven alveolar hypoventilation disorders, hypoxia driven sleep-disordered breathing or chronic exposure to high altitude; pulmonary hypertension associated with development abnormalities; and pulmonary hypertension due to thromboembolic obstruction of distal pulmonary arteries.

The term "effective amount" refers to a therapeutically effective amount of the compound of formula 1 for the preventive or curative treatment of pulmonary hypertension. In case of a combination therapy the term "effective amount" refers to the sum of the amounts of the combination partners, which is therapeutically effective for the preventive or curative treatment of pulmonary hypertension.

Any agent, activator, inhibitor, or compound described herein may be administered systemically, orally, by inhalation, parenteral, nasally, vaginally, rectally, sublingually, topically, or any combination thereof. The agent, activator, inhibitor, or compound may be formulated as a capsule, tablet, an elixir, a suspension, a dry powder, an aerosol, a syrup, or any combination thereof.

As pharmaceutically acceptable auxiliaries, any auxiliaries known to be suitable for preparing pharmaceutical compositions (formulations) can be used. Examples thereof include, but are not limited to, solvents, excipients, dispersants, emulsifiers, solubilizers, gel formers, ointment bases, antioxidants, preservatives, stabilizers, carriers, fillers, binders, thickeners, complexing agents, disintegrating agents, buffers, permeation promoters, polymers, lubricants, coating agents, propellants, tonicity adjusting agents, surfactants, colorants, flavorings, sweeteners and dyes. In particular, auxiliaries of a type appropriate to the desired formulation and the desired mode of administration are used.

In general, rottlerin or a pharmaceutically acceptable salt thereof or one or more of its derivatives or a pharmaceutically acceptable salt thereof and/or the optional compound(s) for combination treatment of this invention will be administered in a therapeutically effective amount by any of the accepted modes of administration for agents and compounds that serve similar utilities. The actual amount of rottlerin or a pharmaceutically acceptable salt thereof or one or more of its derivatives or a pharmaceutically acceptable salt thereof and/or the optional compound(s) for combination treatment of this invention will depend upon numerous factors such as the severity of the disease to be treated, the age and relative health of the subject, the potency of the compound used, the route and form of administration, and other factors. Rottlerin or a pharmaceutically acceptable salt thereof or one or more of its derivatives or a pharmaceutically acceptable salt thereof and/or the optional compound(s) for combination treatment can be administered more than once a day, preferably once or twice a day. Therapeutically effective amounts of rottlerin or a pharmaceutically acceptable salt thereof or one or more of its derivatives or a pharmaceutically acceptable salt thereof and/or the optional compound(s) for combination treatment may range from approximately 0.001 mg to 10 g per kilogram body weight of the subject per day.

Voltage-gated potassium channels of the KCNQ (Kv7) family are functional key players in excitable and epithelial tissues and control and determine the membrane potential of cells accordingly. Drugs can cause both membrane depolarization and hyperpolarization and thus can control the excitability of cells. As a consequence regulating, treating or preventing membrane excitability or a hyperexcitability disorder with such drugs is well possible.

Potassium channels are central to the regulation of pulmonary vascular tone. The vasoconstrictor effect of agents causing membrane depolarization and vasodilation by drugs causing hyperpolarization illustrate the importance of membrane potential for pulmonary arteries (PA) function. The smooth muscle cells of pulmonary artery display a background K⁺ conductance with biophysical properties resembling those of KCNQ4 (Kv7.4) potassium channels. Activation of Kv7 with the unspecific Kv7 activators retigabine and flupirtine, was shown to relax pulmonary arteries [Gurney AM, Joshi S, Manoury B. KCNQ potassium channels: new targets for pulmonary vasodilator drugs? Adv Exp Med Biol. 2010;661:405-17; Joshi S, Sedivy V, Hodyc D, Herget J, Gurney AM. KCNQ modulators reveal a key role for KCNQ potassium channels in regulating the tone of rat pulmonary artery smooth muscle. J Pharmacol Exp Ther. 2009;329(1):368-76.]. The pulmonary vasoconstrictor effect of KCNQ (Kv7) blockers is a potentially serious side effect, but the pulmonary vasodilator effect of KCNQ (Kv7) activators is useful in the treatment of pulmonary hypertension.

However, the lack of specificity of the used KCNQ (Kv7) activators render these compounds not suited for a pharmacological therapy in human.

The inventor has made the surprising discovery that rottlerin specifically activates members of the KCNQ (Kv7) channel family thus can be useful in the treatment of pulmonary hypertension and related diseases and disorders. The shown high potency activation by rottlerin on Kv7.4 provides sufficient selectivity to allow for therapeutic intervention. As rottlerin does not act on the neuronal channels Kv7.2, Kv7.3 and Kv7.5 no neuronal side effects would be expected. As rottlerin is about 30-fold more potent on Kv7.4 that on the cardiac and epithelial Kv7.1/KCNE1 the pharmacological split can be expected sufficient enough to prevent cardiac or epithelial side effects.

### Experimental results:

Heterologous expression of human KCNQ1-5 (Kv7.1-7.5) in *Xenopus laevis* oocytes resulted in characteristic voltage dependent K⁺-selective channel currents.

Rottlerin was applied at different concentrations to the expressed Kv7 channels. In the cases of Kv7.2, Kv7.3 and Kv7.5 no significant effect of the compound was detected. However, on a) Kv7.1, b) Kv7.1/KCNE1 and c) Kv7.4 significant robust activation of currents was detected.

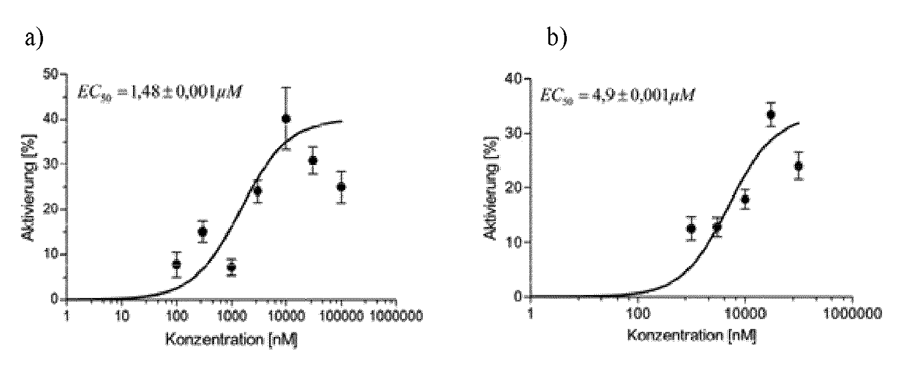

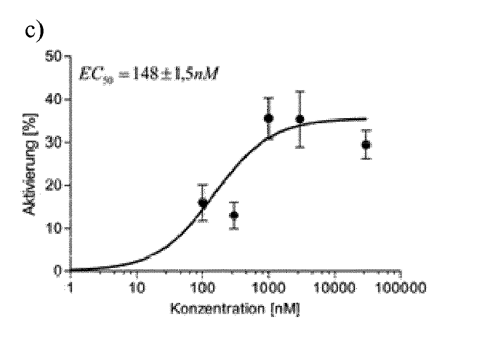

The here presented data prove rottlerin as a naturally product surprisingly acing as very potent selective and novel Kv7.4 activator (EC₅₀ = 148 nM). In addition, the compound activates with lower activity Kv7.1 (EC₅₀ = 1480 nM) and Kv7.1/KCNE1 channels (EC₅₀ = 4900 nM).

## Claims

1. A pharmaceutical composition comprising rottlerin or a pharmaceutically acceptable salt thereof or one or more of its derivatives or a pharmaceutically acceptable salt thereof said derivative having the formula 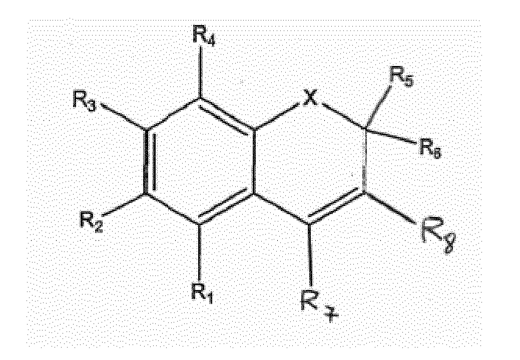 wherein **X** is selected from the group consisting of CH₂, O, N, P, and S; **R1** and **R3** are selected from the group consisting of H, OH, NH₂, SH, SO, S-NH-R, NH-R (R selected from the group consisting of alkyl, alkenyl, alkinyl, aryl, phenyl, benzyl, ester, ether, carbonyl, carboxyl, F, Cl, Br, I, H), alkyl, alkenyl, alkinyl, aryl, phenyl, benzyl, ester, ether, carbonyl, carboxyl, F, Cl, Br, I and H; **R2** is selected from the group consisting of ethanone, acetyl, alkyl, alkenyl alkinyl, aryl, phenyl, benzyl, ester ether, carbonyl, carboxyl, F, Cl, Br, I and H; **R4** is CO-[(E)CHCH]n-Ph, CN-[(E)CHCH]n-Ph, or COOZ, wherein Z is selected from the group consisting of alkenyl, aryl, and alkyl, alkyl, alkenyl, alkinyl, aryl, phenyl, benzyl, ester, ether, carbonyl, carboxyl, F, Cl, Br, I and H; **R5, R6, R7 and R8** are selected from the group consisting of methyl, H, P, OH, NH₂, SH, SO, S-NH-R, NH-R (R selected from the group consisting of alkyl, alkenyl, alkinyl, aryl, phenyl, benzyl, ester, ether, carbonyl, carboxyl, F, Cl, Br, I, H), alkyl, alkenyl, alkinyl, aryl, phenyl, benzyl, ester, ether, carbonyl, carboxyl, F, Cl, Br, I and H, for use in a method of preventing or treating pulmonary hypertension.

2. A pharmaceutical composition comprising rottlerin or a pharmaceutically acceptable salt thereof or one or more of its derivatives according to the formula 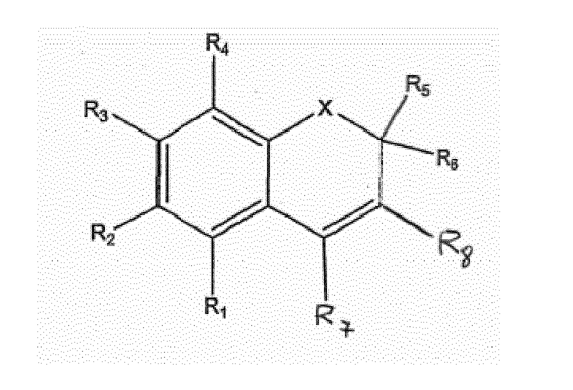 wherein **X** is selected from the group consisting of CH₂, O, N, P, and S; **R1** and **R3** are selected from the group consisting of H, OH, NH₂, SH, SO, S-NH-R, NH-R (R selected from the group consisting of alkyl, alkenyl, alkinyl, aryl, phenyl, benzyl, ester, ether, carbonyl, carboxyl, F, Cl, Br, I, H), alkyl, alkenyl, alkinyl, aryl, phenyl, benzyl, ester, ether, carbonyl, carboxyl, F, Cl, Br, I and H; **R2** is selected from the group consisting of ethanone, acetyl, alkyl, alkenyl alkinyl, aryl, phenyl, benzyl, ester ether, carbonyl, carboxyl, F, Cl, Br, I and H; **R4** is CO-[(E)CHCH]n-Ph, CN-[(E)CHCH]n-Ph, or COOZ, wherein Z is selected from the group consisting of alkenyl, aryl, and alkyl, alkyl, alkenyl, alkinyl, aryl, phenyl, benzyl, ester, ether, carbonyl, carboxyl, F, Cl, Br, I and H; **R5, R6, R7 and R8** are selected from the group consisting of methyl, H, P, OH, NH₂, SH, SO, S-NH-R, NH-R (R selected from the group consisting of alkyl, alkenyl, alkinyl, aryl, phenyl, benzyl, ester, ether, carbonyl, carboxyl, F, Cl, Br, I, H), alkyl, alkenyl, alkinyl, aryl, phenyl, benzyl, ester, ether, carbonyl, carboxyl, F, Cl, Br, I and H, for use in regulating, treating or preventing membrane excitability or a hyperexcitability disorder.

3. The composition of claim 2 where the disorder is a skin disease selected from the group consisting of neurodermatitis or atopic eczema.

4. The composition of claim 1, 2 or 3, further comprising at least one further active pharmaceutical ingredient selected from the group consisting of prostaglandines, phosphodiesterase type 5 inhibitors, endothelium antagonists, calcium channel blockers, selective serotonin reuptake inhibitors (SSRIs), a pulmonary vasodilators, inhibitors of platelet aggregation, platelet derived growth factors, and diuretics, where the at least one further active pharmaceutical ingredient is administered before, after, or at the same time as administering rottlerin or a pharmaceutically acceptable salt thereof or one or more of its derivatives or a pharmaceutically acceptable salt thereof.

5. The composition of claim 4, wherein
• the prostaglandine is selected from the group consisting of alprostadil, epoprostenol, iloprost, and treprostinil;
• the phosphodiesterase type 5 inhibitor is selected from the group consisting of avanafil, enoximon, icariin, mirodenafil, milrinon, sildenafil, sildenafil citrate, tadalafil, vardenafil, and udenafil;
• the endothelium antagonist is selected from the group consisting of ambrisentan, atrasentan, BQ-123, sitaxentan, zibotentan, bosentan, tezosentan, and BQ-788;
• the calcium channel blocker is selected from the group consisting of amlodipine, aranidipine, azelnidipine, barnidipine, benidipine, cilnidipine, clevidipine, isradipine, efonidipine, felodipine, lacidipine, lercanidipine, manidipine, nicardipine, nifedipine, nilvadipine, nimodipine, nisoldipine, nitrendipine, and pranidipine;
• the selective serotonin reuptake inhibitor is selected from the group consisting of citalopram, dapoxetine, escitalopram, fluoxetine, fluvoxamine, indalpine, paroxetine, sertraline, vilazodone, and zimelidine;
• the pulmonary vasodilator is selected from the group consisting of dihydralazine mesylate, dihydralazine sulfate, minoxidile, nitric oxide, and sodium nitroprusside;
• the inhibitor of platelet aggregation is selected from the group consisting of cyclooxygenase inhibitors like acetylsalicylic acid; adenosine diphosphate (ADP) receptor inhibitors like clopidogrel, prasugrel, ticagrelor, ticlopidine; phosphodiesterase inhibitors like cilostazol; glycoprotein IIB/IIIA inhibitors like abciximab, eptifibatide, tirofiban; adenosine reuptake inhibitors like dipyridamole; thromboxane inhibitors (thromboxane synthase inhibitors or thromboxane receptor antagonists) like terutroban;
• the platelet derived growth factor is selected from the group consisting of PDGFA, PDGFB, PDGFC, PDGFD, PDGFRα, and PDGFRβ;
• the diuretic is selected from the group consisting of high ceiling loop diuretics like bumetanide, ethacrynic acid, furosemide, torsemide; thiazides such as hydrochlorothiazide; carbonic anhydrase inhibitors like acetazolamide, methazolamide; potassium-sparing diuretics like aldosterone antagonists (such as spironolactone, potassium canreonate) and epithelial sodium channel blockers (such as amiloride, triamterene); calcium-sparing diuretics; osmotic diuretics like mannitol, isosorbide; and low ceiling diuretics.

6. The composition of one or more of the preceding claims, wherein the treatment systemic, oral, inhalation, parenteral (e.g., subcutaneous, intravenous, bolus injection, intramuscular, or intraarterial), mucosal (e.g., nasal, sublingual, vaginal, buccal, or rectal), transdermal and topical administration.

7. The composition of one or more of the preceding claims, wherein the composition is a formulation selected from the group consisting of a tablets, film-coated tablets; caplets; capsules, such as soft elastic gelatin capsules; cachets; troches; lozenges; dispersions; suppositories; ointments; cataplasms (poultices); pastes; powders; dressings; creams; plasters; implants; solutions; patches; aerosols (e.g., nasal sprays or inhalers); gels; liquid dosage forms suitable for oral or mucosal administration to a patient, including suspensions (e.g., aqueous or nonaqueous liquid suspensions, oil-in-water emulsions, or a water-in-oil liquid emulsions), solutions, and elixirs or syrups; liquid dosage forms suitable for parenteral administration to a patient; and sterile solids (e.g., crystalline or amorphous solids) that can be reconstituted to provide liquid dosage forms suitable for parenteral administration to a patient.

8. The composition of one or more of the preceding claims wherein the dosage form is an immediate release, modified release (prolonged, delayed, extended release) or depot composition.

9. The composition of one or more of the preceding claims, wherein the composition is administered once a day, once a week, once a month or once in three months.

10. The composition of one or more of the preceding claims, wherein the composition is administered more than once a day, preferably once or twice a day.

11. The composition of claim 10 where the route of administration is topical or transdermal in a formulation selected from the group consisting of a cream, gel and ointment.

12. The composition of one or more of the preceding claims wherein the composition comprises therapeutically effective amount(s) of rottlerin or a pharmaceutically acceptable salt thereof or one or more of its derivatives or a pharmaceutically acceptable salt thereof and/or the optional compound(s) for combination treatment in the range from 0.001 mg to 10 g per kilogram body weight of the subject per day.
